# EUROPEAN PATENT APPLICATION

(11) **EP 4 722 236 A1**
(43) Date of publication of application: **08.04.2026**
(21) Application number: 24810208.9
(22) Date of filing: 08.05.2024
(51) Int. Cl.: C07K 14/62, A61K 38/28, A61P 3/10

(54) **INSULIN DERIVATIVE AND USE THEREOF**

(30) Priority: 24.05.2023 CN 202310588063
(71) Applicant: The United Bio-Technology (Hengqin) Co., Ltd., Zhuhai, Guangdong 519031 (CN)
(72) Inventor: HUANG, Liang, Zhuhai, Guangdong 519031 (CN); XIE, Xin, Zhuhai, Guangdong 519031 (CN); CAO, Chunlai, Zhuhai, Guangdong 519031 (CN); ZHOU, Cui, Zhuhai, Guangdong 519031 (CN); HE, Xiuyi, Zhuhai, Guangdong 519031 (CN); DENG, Huixing, Zhuhai, Guangdong 519031 (CN); LIU, Xiaoxiao, Zhuhai, Guangdong 519031 (CN)
(74) Representative: Kröncke, Rolf
(86) International application number: PCT/CN2024/091739
(87) International publication number: WO 2024/239961

(57) **Abstract**

The present invention relates to the field of medicine and specifically provides insulin derivatives of formula (I) or salts or solvates thereof:

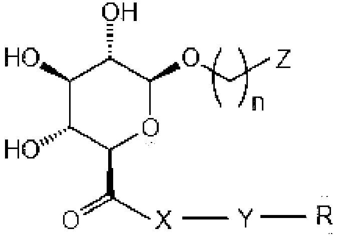

The insulin derivative of formula (I) according to the present invention has better efficacy or effects, prolonged duration of action, and excellent bioavailability and safety.

## Description

### TECHNICAL FIELD

The present invention relates to the pharmaceuticals field, and specifically provides novel insulin derivatives, salts or solvates thereof, pharmaceutical compositions, and uses thereof.

### BACKGROUND ART

Insulin (INS) is a peptide hormone secreted by pancreatic β-cells in the pancreas, its physiological effect is promoting cellular glucose intake, enhancing glycogen synthesis, and inhibiting gluconeogenesis, thereby maintaining normal blood glucose levels and thereby modulating the metabolism of carbohydrates, lipids, and proteins. Structurally, insulin is a heterodimer consisting of two peptide chains with 21 and 30 amino acids, respectively, linked by two interchain disulfide bonds, wherein the A-chain contains an intrachain disulfide bond. The specific amino acid sequences are as follows:
A-chain:
   Gly-Ile-Val-Glu-Gln-Cys-Cys-Thr-Ser-Ile-Cys-Ser-Leu-Tyr-Gln-Leu-Glu-Asn-Tyr-Cys-Asn (SEQ ID NO: 1)
B-chain:
   Phe-Val-Asn-Gln-His-Leu-Cys-Gly-Ser-His-Leu-Val-Glu-Ala-Leu-Tyr-Leu-Val-Cys-Gl y-Glu-Arg-Gly-Phe-Phe-Tyr-Thr-Pro-Lys-Thr (SEQ ID NO:2)

In the case of diabetic patients, insulin fails to function properly due to insulin deficiency, insulin resistance, and loss of β-cell function, thereby glucose in the blood cannot be utilized, leading to elevated blood glucose levels and hyperglycemia. Eventually, glucose is excreted through urine, contributing to the development of various complications. Therefore, insulin therapy is necessary for patients with abnormal insulin production (Type I) or insulin resistance (Type II), which can regulate blood glucose levels to normal ranges through insulin administration.

Currently available insulin products are primarily categorized into five types: rapid-acting insulin, short-acting insulin, intermediate-acting insulin, long-acting insulin, and combination/pre-mixed insulin. However, these insulin products have relatively short duration of action and require subcutaneous injection at least once daily, which causes patients to suffer from various injection-related discomforts. Therefore, people are committed to developing insulin analogues with superior efficacy, prolonged duration of action, and reduced injection frequency to enhance patient compliance.

WO2018109162A1 discloses Icodec, an ultra-long-acting insulin analogue administered once weekly, which is currently undergoing clinical trials in multiple countries worldwide. Its structural formula is as follows:

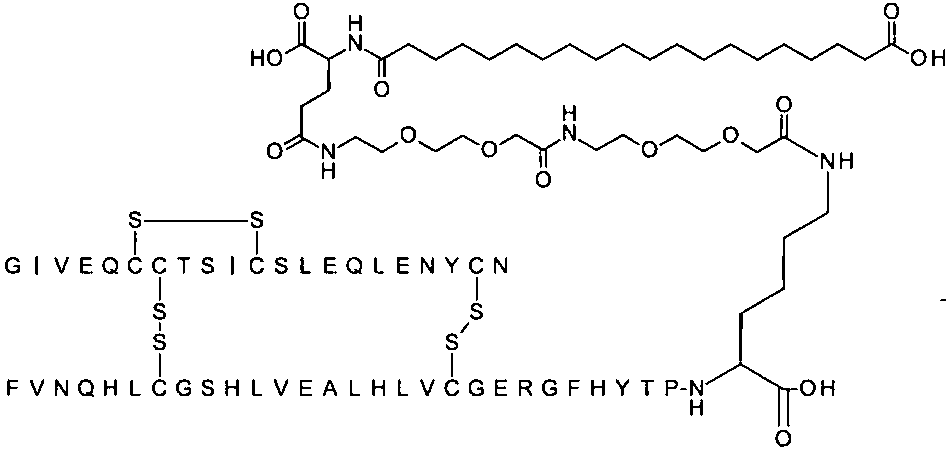

CN 105061601A discloses an insulin conjugate of immunoglobulin fragments, which exhibits a longer duration of action compared to conventional unmodified insulin.

CN101573133B and WO2009/010428 disclose PEG (PEGylated) extended insulin, which exhibits a longer duration of action compared to conventional unmodified insulin.

Therefore, there remains a prevailing demand in the current market for insulin analogues with better drug effect, prolonged duration of action, and reduced administration frequency.

### SUMMARY

In view of the current state of the art, the objective of the present invention is to provide novel insulin derivatives which exhibit better drug effect or efficiency, longer duration of action, as well as favorable bioavailability and safety.

The present invention provides an insulin derivative which has the following Formula (I): 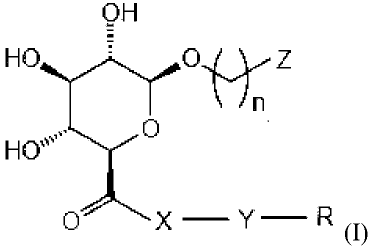 wherein,
Z is CH₃ or COOH;
n is 17,18,19,20 or 21;
X is γ-Glu or absent;
Y is -NH-(CH₂)₂-O-(CH₂)₂-O-CH₂-CO-NH-(CH₂)₂-O-(CH₂)₂-O-CH₂-CO-,or absent;
R is the insulin parent body, wherein the ε-amino group of the lysine side chain at position 29 of the B-chain is connected to Y via an amide bond, the insulin parent body is selected from: A14E, B16H, B25H, desB30 human insulin; A14E, B16H, B25H, desB27, desB30 human insulin.

In the present invention,

The A-chain of A14E, B16H, B25H, desB30 human insulin is as follows:

Gly-Ile-Val-Glu-Gln-Cys-Cys-Thr-Ser-Ile-Cys-Ser-Leu-Glu-Gln-Leu-Glu-Asn-Tyr-Cys
-Asn(SEQ ID NO.3);

The B-chain of A14E, B16H, B25H, desB30 human insulin is as follows: Phe-Val-Asn-Gln-His-Leu-Cys-Gly-Ser-His-Leu-Val-Glu-Ala-Leu-His-Leu-Val-Cys-Gly-Glu-Arg-Gly-Phe-His-Tyr-Thr-Pro-Lys (SEQ ID NO.4).

In the present invention,

The A-chain of A14E, B16H, B25H, desB27, desB30 human insulin is as follows:

Gly-Ile-Val-Glu-Gln-Cys-Cys-Thr-Ser-Ile-Cys-Ser-Leu-Glu-Gln-Leu-Glu-Asn-Tyr-Cys
-Asn(SEQ ID NO.5);

The B-chain of A14E, B16H, B25H, desB30 human insulin is as follows: 
Phe-Val-Asn-Gln-His-Leu-Cys-Gly-Ser-His-Leu-Val-Glu-Ala-Leu-His-Leu-Val-Cys-G
ly-Glu-Arg-Gly-Phe- His -Tyr- Pro-Lys(SEQ ID NO.6).
 Phe-Val-Asn-Gln-His-Leu-Cys-Gly-Ser-His-Leu-Val-Glu-Ala-Leu-His-Leu-Val-Cys-G ly-Glu-Arg-Gly-Phe- His -Tyr- Pro-Lys(SEQ ID NO.6).

In the present application, as one of the embodiments, the insulin derivative of Formula (I) is selected from:
Compound 1: A14E, B16H, B25H, B29K(N^{ε}-[1-(19-Carboxyl-nonadecyloxy)-β-D-glucuronyl]-[2-(2-{2-[2-(2-Aminoethoxy)ethoxy ]acetylamino}ethoxy)ethoxy]acetyl), desB30 human insulin;
Compound 2: A14E, B16H, B25H, B29K(N^{ε}-[1-(19-Carboxyl-nonadecyloxy)-β-D-glucuronyl]-γGlu-[2-(2-{2-[2-(2-Aminoethoxy) ethoxy]acetylamino}ethoxy)ethoxy]acetyl), desB30 human insulin;
Compound 3: A14E, B16H, B25H, B29K(N^{ε}-[1-(19-Carboxyl-nonadecyloxy)-β-D-glucuronyl]-[2-(2-{2-[2-(2-Aminoethoxy)ethoxy ]acetylamino}ethoxy)ethoxy]acetyl), desB27, desB30 human insulin;
Compound 4: A14E, B16H, B25H, B29K(N^{ε}-[1-(19-Carboxyl-nonadecyloxy)-β-D-glucuronyl]-γGlu-[2-(2-{2-[2-(2-Aminoethoxy) ethoxy]acetylamino}ethoxy)ethoxy]acetyl), desB27, desB30 human insulin;
In the present application, as one of the embodiments, the insulin derivative of Formula (I) is selected from:
   Compound 2: A14E, B16H, B25H, B29K(N^{ε}-[1-(19-Carboxyl-nonadecyloxy)-β-D-glucuronyl]-γGlu-[2-(2-{2-[2-(2-Aminoethoxy) ethoxy]acetylamino}ethoxy)ethoxy]acetyl), desB30 human insulin;
   In another aspect, the present invention provides pharmaceutical compositions comprising an effective amount of the insulin derivative of Formula (I), or salts or solvates thereof, and one or more pharmaceutically acceptable excipient, diluents, carriers, or vehicle.

In the present application, as one of the embodiments, the pharmaceutical composition is injection or lyophilized powder, tablet, pill, lozenge, soft capsule, hard capsule, granule, powder, solution, microneedle, suspension, or syrup.

In the present application, as one of the embodiments, the pharmaceutical composition is in the form of microcapsule, microsphere, nanoparticle, or liposome.

In the present application, as one of the embodiments, the pharmaceutical composition is intended for oral administration, inhalation administration, transdermal administration, or parenteral administration, wherein the parenteral administration is selected from intraperitoneal, intramuscular, intra-arterial, intravenous, subcutaneous, or intradermal injection.

In the present application, as one of the embodiments, the pharmaceutical composition is administered at a frequency of at least once daily, once weekly, or once monthly.

The present invention provides the use of the insulin derivative of Formula (I), or salts or solvates thereof, in the preparation of a medicament for the treatment, prevention, or alleviation of diseases including diabetes, type 1 diabetes, type 2 diabetes, impaired glucose tolerance, hyperglycemia, dyslipidemia, obesity, and related disorders.

The present invention provides a combination of the insulin derivative of Formula (I), or salts or solvate thereof, with other drugs for treating the same or related disorders. The other drugs include, but are not limited to, metformin agents, sulfonylureas, SGLT-1/2 inhibitors, DPP-4 inhibitors, insulin and analogs thereof, GLP-1 and analogs thereof, GCG and analogs thereof, GIP and analogs thereof, FGF-21 and analogs thereof, or multi-target drugs among the above drugs, or two or more of them.

The insulin derivative of Formula (I) according to the present invention exhibits better drug effect or efficiency, longer duration of action, as well as favorable bioavailability and safety.

### BRIEF DESCRIPTION OF THE DRAWINGS

Figs. 1a and 1b show the blood glucose-lowering effects of the solvent, Icodec, and Compound 2 administered subcutaneously in normal SD rats in Experimental Example 4.
Figs. 2a and 2b show the blood glucose-lowering effects of the solvent, Icodec, Compound 3 and Compound 4 administered subcutaneously in normal SD rats in Experimental Example 4.
Figs. 3a and 3b show the blood glucose-lowering effects of intravenous administration of the solvent, Icodec, and Compound 2 in normal beagle dogs in Experimental Example 5.

### DETAILED DESCRIPTION

The following examples and experimental examples are provided to further illustrate the present invention, but are not in any way intended to limit the effective scope of the present invention.

### Example 1: Preparation of Compound 2

The molecular formula and structural formula of Compound 2 are as follows:
A14E, B16H, B25H, B29K (N^{ε}-[1-(19-carboxyl-nonadecyloxy)-β-D-glucuronyl]-γGlu-[2-(2-{2-[2-(2-aminoethoxy) ethoxy]acetylamino}ethoxy)ethoxy]acetyl), desB30 human insulin;

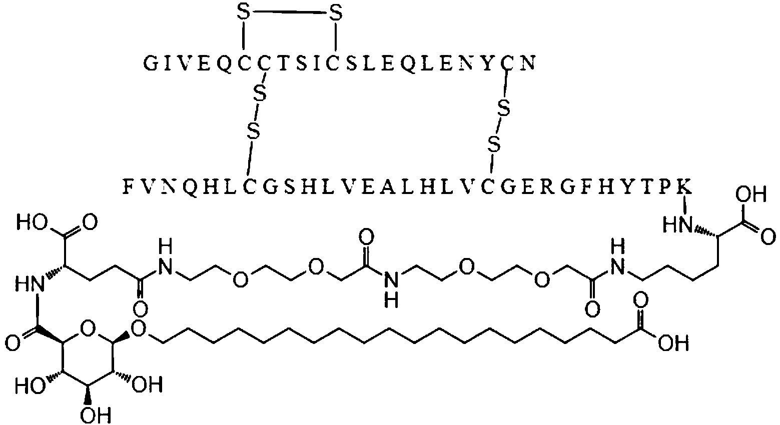

### Preparation of Fatty Acid Derivatives

### (1) Preparation of Methyl 1,2,3,4-Tetraacetoxy-D-glucopyranuronate

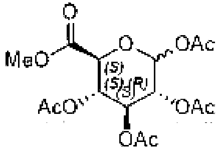

To a solution of D-glucuronolactone (CAS 32449-92-6) (100 g, 567 mmol, 1.00 equiv) in MeOH (300 mL) was added NaOH (567 mg, 14.1 mmol, 0.025 equiv),. The mixture was stirred at 20°C for 16 hours. The mixture was concentrated under vacuum and Pyridine (179 g, 2.27 mol, 183 mL, 4.00 equiv), Ac₂O (289 g, 2.84 mol, 265 mL, 5.00 equiv) was added at 5°C. The resulting mixture was stirred at 20°C for 3 hours. The reaction mixture was cooled to 0°C and filtered to give the filter cake. The filter cake was triturated with ethanol (100 mL) at 20°C for 10 hours. The mixture was then cooled to 0°C, filtered, and the filter cake was concentrated under reduced pressure to give methyl 1,2,3,4-Tetraacetoxy-D-glucopyranuronate (43.9 g) as a white solid.

¹H NMR:400 MHz, CDCl₃. δ: 5.77 (d, J = 7.6 Hz, 1H), 5.32 - 5.23 (m, 2H), 5.17 - 5.13 (m, 1H), 4.18 (d, J = 9.2 Hz, 1H), 3.75 (s, 3H), 2.12 (s, 3H), 2.04 - 2.04 (m, 9H).

### (2) Preparation of Methyl 1-bromo-2,3,4-triacetoxy-α-D-glucopyranuronate

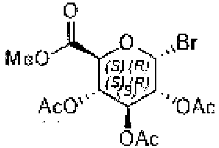

At 0°C, methyl 1,2,3,4-Tetraacetoxy-D-glucopyranuronate (43.9 g, 116 mmol, 1.00 equiv) was added into a solution of hydrogen bromide in acetic acid (150 mL, 33% wt). The mixture was stirred at 20°C for 3 hours. The reaction mixture was concentrated under reduced pressure to give a residue. The residue was triturated with ethanol (50 mL) at 20°C for 10 hours, cooled to 0°C, and filtered. The filter cake was concentrated under reduced pressure to give methyl 1-bromo-2,3,4-triacetoxyl-α-D-glucopyranuronate (28.5 g) as a white solid.

¹H NMR: 400 MHz, CDCl₃. δ: 6.64 (d, J = 4.0 Hz, 1H), 5.62 (t, J = 9.6 Hz, 1H), 5.27 - 5.22 (m, 1H), 4.86 (dd, J1 = 4.0 Hz, J2 = 10.0 Hz, 1H), 4.59 (d, J = 10.4 Hz, 1H), 3.77 (s, 3H), 2.10 (s, 3H), 2.06 (d, J = 2.5 Hz, 6H).

### (3) Preparation of tert-Butyl 20-Hydroxyicosanoate

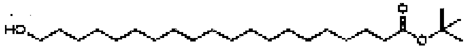

Mono-tert-butyl eicosanedioate (180 g, 451 mmol, 1.00 equiv) and N-methylmorphine (54.8 g, 541 mmol, 1.10 equiv) were added to tetrahydrofuran solution (1000 mL). The mixture was cooled to -10°C under nitrogen, and isobutyl chloroformate (61.6 g, 451 mmol, 1.10 equiv) was added. The mixture was then stirred at -10°C for 1 hour and filtered. At 0°C, sodium borohydride (25.6 g, 677 mmol, 1.50 equiv) was added to the filtrate under nitrogen. After the reaction was complete, the mixture was filtered, the filtrate is concentrated under the reduced pressure to give residues. Tert-butyl 20-hydroxyicosanoate (29.0 g) was given as a white solid by purification with column chromatography (silicon dioxide, petroleum ether: ethyl acetate = 100:1:1, Rf = 0.27).

¹H NMR: 400 MHz, CDCl₃.δ: 3.64 (t, J = 6.8 Hz, 2H), 2.20 (t, J = 7.6 Hz, 2H), 1.60 - 1.53 (m, 4H), 1.44 (s, 9H), 1.41 - 1.25 (m, 31H).

### (4) Preparation of Methyl 1-((20-(tert-Butoxyl)-20-oxoicosyl)oxy)-2,3,4-triaceto xy-β-D-glucopyranuronate

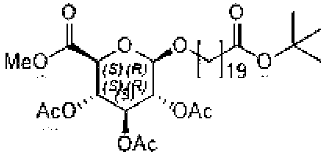

Tert-butyl 20-hydroxyicosanoate (29.0 g, 75.5 mmol, 2.00 equiv), silver oxide (43.7 g, 188 mmol, 5.00 equiv), iodine (17.2 g, 67.9 mmol, 1.80 equiv), and calcium sulfate (102 g, 755 mmol, 20.00 equiv) were added to chloroform solution (150 mL). The mixture was stirred under nitrogen at 20°C for 15 minutes. A solution of methyl 1-bromo-2,3,4-triacetoxy-α-D-glucopyranuronate (15.0 g, 37.7 mmol, 1.00 equiv) in chloroform solution (50 mL) was then added. The resulting mixture was stirred at 30°C for 18 hours. After the reaction, the filtrate is filtered and concentrated under reduced pressure to give the residue. The residue is purified by column chromatography (silicon dioxide, petroleum ether:ethyl acetate = 100:1 to 1:1, petroleum ether: ethyl acetate=3:1) (Rf=0.30) to give methyl 1-((20-(tert-Butoxy)-20-oxoicosyl)oxy)-2,3,4-triacetoxy-β-D-glucopyranuronate(9.28 g) as a white oily substance.

LC-MS: m/z=723.0[M+Na]⁺, consistent with theoretical values.

¹H NMR: 400 MHz, CDCl₃.δ: 5.30 - 5.22 (m, 2H), 5.03 - 4.98 (m, 1H), 4.54 ( d, J = 7.6 Hz, 1H), 4.04 - 4.02 (m, 1H), 3.91 - 3.88 (m, 1H), 3.76 (s, 3H), 3.48 - 3.4 6 (m, 1H), 2.20 (t, J = 7.6 Hz, 2H), 2.03 (d, J = 7.2 Hz, 9H), 1.60 - 1.53 (m, 5H), 1. 44 (s, 9H), 1.27 - 1.25 (m, 30H).

### (5) Preparation of 1-((20-(tert-Butoxy)-20-oxoicosyl)oxy)-β-D-glucopyranuronic acid

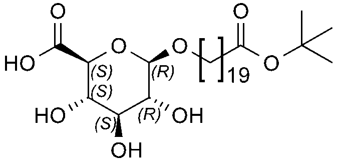

Methyl-((20-(tert-Butoxy)-20-oxoicosyl)oxy)-2,3,4-triacetoxyl-β-D-glucopyranuronate (9.28 g, 13.2 mmol, 1.00 equiv) and sodium methoxide (35.7 mg, 662 µmol, 0.05 equiv) were added to methanol solution (50 mL). The mixture was stirred at 20°C for 5 hours. Lithium hydroxide monohydrate (555 mg, 13.2 mmol, 1.00 equiv) was then added. The mixture was stirred at 20°C for 17 hours. The reaction mixture was concentrated under reduced pressure to give 1-((20-(tert-butoxy)-20-oxoicosyl)oxy)-β-D-glucopyranuronic acid (11.3 g) as a pale yellow solid.

LC-MS: m/z=599.3[M-H]⁻, consistent with theoretical values.

¹H NMR: 400 MHz, MeOD.δ: 4.28 (d, J = 8.0 Hz, 1H), 3.92 - 3.68 (m, 1H), 3.65 - 3.48 (m, 1H), 3.45 - 3.40 (m, 2H), 3.38 (t, J = 9.2 Hz, 1H), 3.21 (dd, J1 = 7.6 Hz, J2 = 9.1 Hz, 1H), 2.20 (t, J = 7.6 Hz, 2H), 1.64 - 1.54 (m, 4H), 1.44 (s, 9H), 1. 39 - 1.36 (m, 2H), 1.29 (br s, 28H).

### Preparation of Intermediate 1 of Compound 2

(1) Resin preparation: 2-CTC resin (150.0 mmol, 1.00 equiv, substitution: 1.00 mmol/g), Fmoc-Cys(Trt)-OH (150.0 mmol, 1.00 equiv), and DIEA (600 mmol, 4.00 equiv) were added to dichloromethane solution (1500 mL). The mixture was stirred under nitrogen at 20°C for 2 h. Methanol (150.0 mL) was then added, and stirring was continued for an additional 30 min. The resin was washed with N, N-dimethylformamide (1500 mL* 5) and then filtered to give the resin.
(2) Deprotection: The resin-containing solution of N, N-dimethylformamide (1500 mL, containing 20% piperidine) was stirred under nitrogen for 30 min. The resin was then washed with N, N-dimethylformamide (1500 mL* 5) and filtered to give the resin.
(3) Coupling: Fmoc-Ile-OH (450 mmol, 3.00 equiv), DIEA (900 mmol, 6.00 equiv), and HBTU (427.5 mmol, 2.85 equiv) were added to a resin-containing solution of N, N-dimethylformamide (1000 mL). The mixture was stirred under nitrogen at 20°C for 30 min. The resin was washed with N, N-dimethylformamide (1500 mL* 5).
(4) Repeat steps (2) to (3) above to perform coupling on the following materials:

**Table 1 Materials for Preparing Intermediate 1 of Compound 2**

| # | **Materials** | **Coupling Agents** |
|---|---|---|
| 1 | Fmoc-Ser(tBu)-OH (3.00 equiv) | HBTU (2.85 equiv) and DIEA (6.00 equiv) |
| 2 | Fmoc-Thr(tBu)-OH (3.00 equiv) | HBTU (2.85 equiv) and DIEA (6.00 equiv) |
| 3 | Fmoc-Cys(Acm)-OH (2.00 equiv) | HBTU (1.90 equiv) and DIEA (4.00 equiv) |
| 4 | Fmoc-Cys(Trt)-OH (3.00 equiv) | HBTU (2.85 equiv) and DIEA (6.00 equiv) |

(5) The resin was washed with methanol (2400 mL*3) and dried under vacuum to give the peptide resin. A cleavage buffer (2400 mL) (92.5% trifluoroacetic acid/2.5% triisopropylsilane/2.5% water/2.5% mercaptopropionic acid) was then added to the flask containing the side-chain protected peptide resin at 20°C, and the mixture was stirred for 2 h. The peptide fragment was precipitated with cold isopropyl ether (10000 mL), filtered and the filter cake was collected. The filter cake then was washed twice with isopropyl ether (50000 mL).
(6) The filter cake was dried under vacuum for 2 hours and then dissolved with N, N-dimethylformamide (DMF) (30.0 L). The solution was maintained at a constant temperature of 20°C, and a 0.1 M methanol-iodine solution was added dropwise until yellow color remains unchanged. After stirring for 2 minutes, a 0.1 M aqueous solution of sodium thiosulfate was added dropwise until the yellow color disappears. The mixture was then lyophilized to give intermediate 1 (180 g) as a white solid.

LC-MS: m/z=920.5[M+H]⁺, consistent with theoretical values.

### Preparation of Intermediate 2 of Compound 2

(1) Resin preparation: 2-CTC resin (70.0 mmol, 1.00 equiv, substitution: 1.00 mmol/g), Fmoc-Gln(Trt)-OH (70.0 mmol, 1.00 equiv), and DIEA (280 mmol, 4.00 equiv) were added separately to the dichloromethane solution. The mixture was stirred for 2 hours at 20°C under nitrogen. Then, methanol (70.0 mL) was added and stirred for another 30 minutes. The resin was washed with N,N-dimethylformamide (700 mL* 5), and filtered to give the resin.
(2) Deprotection: The resin-containing solution of N,N-dimethylformamide (700 mL, containing 20% piperidine) was stirred under nitrogen for 30 min. The resin was then washed with N,N-dimethylformamide (700 mL* 5), and filtered to give the resin.
(3) Coupling: Fmoc-Glu(tBu)-OH (210 mmol, 3.00 equiv), DIEA (420 mmol, 6.00 equiv), and HBTU (199.5 mmol, 2.85 equiv) were added to a resin-containing solution of N,N-dimethylformamide (400 mL). The mixture was stirred at 20°C under nitrogen for 30 min. The resin was washed with N,N-dimethylformamide (700 mL* 5).
(4) Repeat steps (2) to (3) above to perform coupling on the following materials:

**Table 2 Materials for Preparing Intermediate 2 of Compound 2**

| **#** | **Materials** | **Coupling Agents** |
|---|---|---|
| 1 | Fmoc-Val-OH (3.00 equiv) | HBTU (2.85 equiv) and DIEA (6.00 equiv) |
| 2 | Fmoc-Ile-OH (3.00 equiv) | HBTU (2.85 equiv) and DIEA (6.00 equiv) |
| 3 | Boc-Gly-OH (3.00 equiv) | HBTU (2.85 equiv) and DIEA (6.00 equiv) |

(5) The resin was washed with methanol (1000 mL *3) and vacuum-dried to give the peptide resin. Then 1000 mL of cleavage buffer (20% hexafluoroisopropanol/80% dichloromethane) was added to the flask containing the side-chain protected peptide resin at room temperature, with stirring performed twice for 30 minutes each. Intermediate 2 (61.77 g) was given as a white solid by pressure concentration.

LC-MS: m/z=943.7[M+H]⁺, consistent with theoretical values.

### Preparation of Intermediate 3 of Compound 2

(1) Resin preparation: 2-CTC resin (20.0 mmol, 1.00 equiv, substitution:1.00 mmol/g), Fmoc-Asn(Trt)-OH (20.0 mmol, 1.00 equiv), and DIEA (80.0 mmol, 4.00 equiv) were added separately to the dichloromethane solution(150 mL). The mixture was stirred for 2 hours at 20°C under nitrogen. Then, methanol (20.0 mL) was added and stirred for another 30 minutes. The resin was washed with N,N-dimethylformamide (300 mL*5), and filtered to give the resin.
(2) Deprotection: The resin-containing solution of N,N-dimethylformamide (300 mL, containing 20% piperidine) was stirred under nitrogen for 30 min. The resin was then washed with N,N-dimethylformamide (300 mL* 5), and filtered to give the resin.
(3) Coupling: Fmoc-Cys(Trt)-OH (60.00 mmol, 3.00 equiv), DIEA (120 mmol, 12.00 equiv), and HBTU (57.0 mmol, 2.85 equiv) were added to the resin-containing solution of N,N-dimethylformamide (300 mL ). The mixture was stirred at 20°C under nitrogen for 30 min. The resin was washed with N,N-dimethylformamide (300 mL*5).
(4) Repeat steps (2) to (3) above to perform coupling on the following materials:

**Table 3 Materials for Preparing Intermediate 3 of Compound 2**

| **#** | **Materials** | **Coupling Agents** |
|---|---|---|
| 1 | Fmoc-Tyr(tBu)-OH (3.00 equiv) | HBTU (2.85 equiv) and DIEA (6.00 equiv) |
| 2 | Fmoc-Asn(Trt)-OH (3.00 equiv) | HBTU (2.85 equiv) and DIEA (6.00 equiv) |
| 3 | Fmoc-Glu(OtBu)-OH (3.00 equiv) | HBTU (2.85 equiv) and DIEA (6.00 equiv) |
| 4 | Fmoc-Leu-OH (3.00 equiv) | HBTU (2.85 equiv) and DIEA (6.00 equiv) |
| 5 | Fmoc-Gln(Trt)-OH (3.00 equiv) | HBTU (2.85 equiv) and DIEA (6.00 equiv) |
| 6 | Fmoc-Glu(OtBu)-OH (3.00 equiv) | HBTU (2.85 equiv) and DIEA (6.00 equiv) |
| 7 | Fmoc-Leu-OH (3.00 equiv) | HBTU (2.85 equiv) and DIEA (6.00 equiv) |
| 8 | Fmoc-Ser(tBu)-OH (3.00 equiv) | HBTU (2.85 equiv) and DIEA (6.00 equiv) |
| 9 | Intermediate 1 (2.00 equiv) | HBTU (2.00 equiv) and DIEA (2.00 equiv) |
| 10 | Intermediate 2 (3.00 equiv) | HBTU (2.85 equiv) and DIEA (6.00 equiv) |

(5) The resin-containing solution of N, N-dimethylformamide (300 mL, containing 20% piperidine) was stirred under nitrogen for 30 min. The resin was then washed with N,N-dimethylformamide (300 mL* 5), and filtered to give the resin.
(6) The resin was washed with methanol (2400 mL *3) and vacuum-dried to give the peptide resin. Then 400 mL of cleavage buffer solution (95% trifluoroacetic acid/2.5% triisopropylsilane/2.5% water) was added to the flask containing the side-chain protected peptide resin at 20°C and the mixture was stirred for 2 hours. The peptide fragments was precipitated using cold isopropyl ether (2000 mL), and was filtered to collect filter cake. The filter cake was then washed twice with isopropyl ether (1000 mL).
(7) The crude peptide is dried in vacuum for 2 hours, then dissolved in N,N-dimethylformamide, and purified by high-performance liquid chromatography to give intermediate 3 (1.9 g) as a white solid.

LC-MS: m/z=1210.0[M+2H]²⁺, consistent with theoretical values.

**Table 4 Purification Conditions for Compound 2 Intermediate 3**

| Instruments | Gilson GX-281 |
|---|---|
| Mobile Phase | A: 0.075% trifluoroacetic acid/water |
| | B: Acetonitrile |
| Gradient | 0-0%-15 min, 20-50%-50 min, Retention time: 40 min |
| Chromatography Column | Gemini,5µm,C18,110A+luna,C18,10µm,100A |
| Flow Rate | 20 mL/Min |
| Wavelength | 214/254 nm |
| Column Temperature | 50°C |

### Preparation of Intermediate 4 of Compound 2

(1) Resin preparation: 2-CTC resin (2.00 mmol, 1.00 equiv, substitution: 1.00 mmol/g), Fmoc-Lys (Dde)-OH (2.00 mmol, 1.00 equiv), and DIEA (8.00 mmol, 4.00 equiv) were added separately to a solution of dichloromethane (20.0 mL). The mixture was stirred for 2 hours at 20°C under nitrogen. Then, methanol (4.00 mL) was added and stirred for another 30 minutes. The resin was washed with N,N-dimethylformamide (20.0 mL*5), and filtered to give the resin.
(2) Deprotection: The resin-containing solution of N,N-dimethylformamide (20.0 mL, containing 20% piperidine) was stirred under nitrogen for 30 minutes. The resin was then washed with N,N-dimethylformamide (20.0 mL* 5) and filtered to give the resin.
(3) Coupling: Fmoc-Pro-OH (6.00 mmol, 3.00 equiv), DIEA (6.00 mmol, 12.00 equiv), and HBTU (5.70 mmol, 2.85 equiv) were added to a resin-containing solution of N,N-dimethylformamide (10.0 mL). The mixture is stirred under nitrogen at 20°C for 30 minutes. The resin is washed with N, N-dimethylformamide (20.0 mL*5).
(4) Repeat steps (2) to (3) above to perform coupling on the following materials (1-27):

**Table 5 Materials for Preparing Intermediate 4 of Compound 2**

| # | **Materials** | **Coupling agents** |
|---|---|---|
| 1 | Fmoc-Thr(tBu)-OH (3.00 equiv) | HBTU (2.85 equiv) and DIEA (6.00 equiv) |
| 2 | Fmoc-Tyr(tBu)-OH (3.00 equiv) | HBTU (2.85 equiv) and DIEA (6.00 equiv) |
| 3 | Fmoc-His(Trt)-OH (3.00 equiv) | HBTU (2.85 equiv) and DIEA (6.00 equiv) |
| 4 | Fmoc-Phe-OH (3.00 equiv) | HBTU (2.85 equiv) and DIEA (6.00 equiv) |
| 5 | Fmoc-Gly-OH (3.00 equiv) | HBTU (2.85 equiv) and DIEA (6.00 equiv) |
| 6 | Fmoc-Arg(Pbf)-OH (3.00 equiv) | HBTU (2.85 equiv) and DIEA (6.00 equiv) |
| 7 | Fmoc-Glu(OtBu)-OH (3.00 equiv) | HBTU (2.85 equiv) and DIEA (6.00 equiv) |
| 8 | Fmoc-Gly-OH (3.00 equiv) | HBTU (2.85 equiv) and DIEA (6.00 equiv) |
| 9 | Fmoc-Cys(Trt)-OH (3.00 equiv) | HBTU (2.85 equiv) and DIEA (6.00 equiv) |
| 10 | Fmoc-Val-OH (3.00 equiv) | HATU (2.85 equiv) and DIEA (6.00 equiv) |
| 11 | Fmoc-Leu-OH (3.00 equiv) | HATU (2.85 equiv) and DIEA (6.00 equiv) |
| 12 | Fmoc-His(Trt)-OH (3.00 equiv) | HATU (2.85 equiv) and DIEA (6.00 equiv) |
| 13 | Fmoc-Leu-OH (3.00 equiv) | HATU (2.85 equiv) and DIEA (6.00 equiv) |
| 14 | Fmoc-Ala-OH (3.00 equiv) | HATU (2.85 equiv) and DIEA (6.00 equiv) |
| 15 | Fmoc-Glu(OtBu)-OH (3.00 equiv) | HATU (2.85 equiv) and DIEA (6.00 equiv) |
| 16 | Fmoc-Val-OH (3.00 equiv) | HATU (2.85 equiv) and DIEA (6.00 equiv) |
| 17 | Fmoc-Leu-OH (3.00 equiv) | HATU (2.85 equiv) and DIEA (6.00 equiv) |
| 18 | Fmoc-His(Trt)-OH (3.00 equiv) | HATU (2.85 equiv) and DIEA (6.00 equiv) |
| 19 | Fmoc-Ser(tBu)-OH (3.00 equiv) | HATU (2.85 equiv) and DIEA (6.00 equiv) |
| 20 | Fmoc-Gly-OH (3.00 equiv) | HATU (2.85 equiv) and DIEA (6.00 equiv) |
| 21 | Fmoc-Cys(Acm)-OH (2.00 equiv) | HATU (1.90 equiv) and DIEA (4.00 equiv) |
| 22 | Fmoc-Leu-OH (3.00 equiv) | HATU (2.85 equiv) and DIEA (6.00 equiv) |
| 23 | Fmoc-His(Trt)-OH (3.00 equiv) | HATU (2.85 equiv) and DIEA (6.00 equiv) |
| 24 | Fmoc-Gln(Trt)-OH (3.00 equiv) | HATU (2.85 equiv) and DIEA (6.00 equiv) |
| 25 | Fmoc-Asn(Trt)-OH (3.00 equiv) | HATU (2.85 equiv) and DIEA (6.00 equiv) |
| 26 | Fmoc-Val-OH (3.00 equiv) | HATU (2.85 equiv) and DIEA (6.00 equiv) |
| 27 | Boc-Phe-OH (3.00 equiv) | HATU (2.85 equiv) and DIEA (6.00 equiv) |
| 28 | AEEA (3.00 equiv) | HBTU (2.85 equiv) and DIEA (6.00 equiv) |
| 29 | AEEA (3.00 equiv) | HBTU (2.85 equiv) and DIEA (6.00 equiv) |
| 30 | Fmoc-γGlu(OtBu) -OH (3.00 equiv) | HBTU (2.85 equiv) and DIEA (6.00 equiv) |
| 31 | **1-((20-(tert-Butoxy)-20-oxoicosyl)oxy)-β-D -glucopyranuronic acid** | HOAt (2.00 equiv) and DIC (2.00 equiv) |

(5) 3% hydrazine hydrate/N,N-dimethylformamide (20.0 mL) was added, and reacted for 30 min. The resin was washed with N,N-dimethylformamide (20.0 mL*5). Repeat the above steps (2) to (3) to couple materials (28-31).
(6) washing with methanol, drying under vacuum, to give the peptide resin. Then, 150 mL of cleavage buffer solution (92.5% trifluoroacetic acid/2.5% triisopropylsilane/2.5% water/2.5% mercaptopropionic acid) is added, and the mixture is stirred at 20°C for 2 hours. The peptide fragment is precipitated by using cold isopropyl ether (750 mL). filtering and collecting filter cake. It is washed twice with isopropyl ether (400 mL). The crude peptide is dried in vacuum for 2 hours.
(7) Crude peptide (3.0 g), 2,2'-dithiodipyridine (154.7 mg, 2.00 equiva), and DIEA (181.5 mg, 4.00 equiv) were added to dimethyl sulfoxide solution (25.0 mL). The mixture was stirred at 20°C for 10 minutes.
(8) The crude peptide was purified by preparative high-performance liquid chromatography to yield intermediate 4 (1.9 g) as a white solid.

LC-MS: m/z=1095.8[M+4H]⁴⁺, consistent with theoretical values.

**Table 6 Purification Conditions for Intermediate 4 of Compound 2**

| Instruments | Gilson GX-281 |
|---|---|
| Mobile Phase | A: 0.075% trifluoroacetic acid/water |
| | B: acetonitrile |
| Gradient | 0-0%-7 min, 30-50%-50 min, Retention time: 30 min |
| Chromatography Column | Gemini,5um,C18,110A+luna,C18,10um,100A |
| Flow Rate | 20 mL/Min |
| Wavelength | 214/254 nm |
| Column Temperature | 30°C |

### Preparation of Intermediate 5 for Compound 2

Intermediate 3 (243.0 mg, 1.10 equiv), intermediate 4 (400.0 mg, 1.00 equiv), and DIEA (11.80 mg, 1.00 equiv) were added to dimethyl sulfoxide solution (10 mL). The mixture was stirred at 20°C for 10 minutes.

After the reaction, filtration was performed to remove insoluble matter. The crude peptide was purified by high-performance liquid chromatography, yield intermediate 5 (200.5 mg) as a white solid.

LC-MS: m/z=1636.9 [M+4H]⁴⁺, consistent with theoretical values.

**Table 7 Purification Conditions for Intermediate 5 of Compound 2**

| Instruments | Gilson GX-281 |
|---|---|
| Mobile Phase | A: 0.075% trifluoroacetic acid/water |
| | B: acetonitrile |
| Gradient | 5-5%-6 min, 32-50%-28 min, Retention time: 23 min |
| Chromatography Column | Gemini,5um,C18,110A+luna,C18,10um,100A |
| Flow Rate | 20 mL/Min |
| Wavelength | 214/254 nm |
| Column Temperature | 30°C |

### Preparation of Compound 2

Intermediate 5 (200.5 mg, 1.00 equiv), water (7.00 mL), acetonitrile (3.00 mL), hydrochloric acid (0.50 mL), acetic acid (2.00 mL), and iodine (64.6 mg, 10.00 equiv) were mixed. The mixture was stirred at 20°C for 10 minutes.

After the reaction, filtration was performed to remove insoluble matter. The crude peptide was purified using high-performance liquid chromatography, yielding the final product Compound 2 (13.7 mg) as a white solid.

LC-MS: m/z=1636.6[M+4H]⁴⁺, consistent with theoretical values.

**Table 8 Purification Conditions for Compound 2**

| Instruments | Gilson GX-281 |
|---|---|
| Mobile Phase | A: water (containing 0.075% Trifluoroacetic Acid aqueous solution) |
| | B: CH₃CN |
| Gradient | 1%ACN 10min,26-56-50min,Retention time: 30 min |
| Chromatography Column | Gemini,5um, C18,110A+luna,C18,10um,100A |
| Flow Rate | 20 mL/min |
| Wavelength | 214/254 nm |
| Column Temperature | 30°C |

### Example 2: Preparation of Compound 1

### The molecular formula and structural formula of Compound 1 are as follows:

A14E, B16H, B25H, B29K(N^{ε}-[1-(19-Carboxyl-nonadecyloxy)-β-D-glucuronyl]-[2-( 2-{2-[2-(2-Aminoethoxy)ethoxy]acetylamino}ethoxy)ethoxy]acetyl), desB30 human insulin

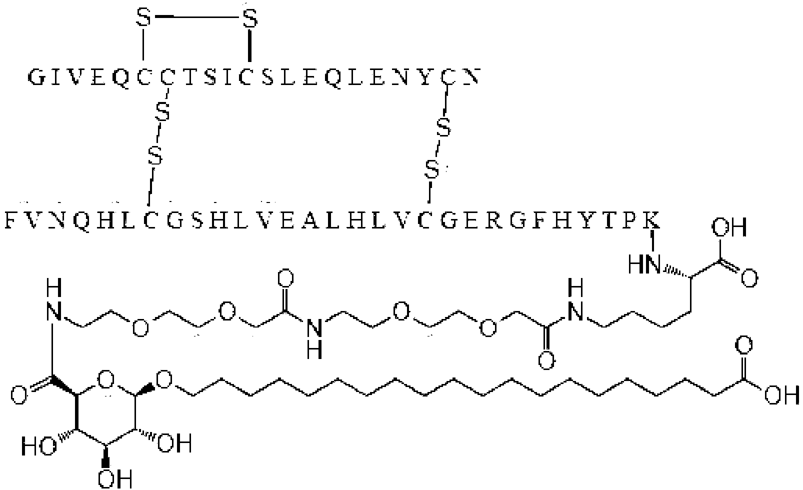

Compound 1 was prepared using the method described in Example 1, with the Fmoc-γGlu(OtBu)-OH removed as listed in Table 5. All other materials and preparation methods remained unchanged.

LC-MS: m/z=1604.3 [M+4H]⁴⁺, consistent with theoretical values.

### Example 3: Preparation of Compound 3

### The molecular formula and structural formula of Compound 3 are as follows:

A14E, B16H, B25H, B29K (N^{ε}-[1-(19-Carboxyl-nonadecyloxy)-β-D-glucuronyl]-[2-(2-{2-[2-(2-Aminoethoxy)ethoxy]acety lamino}ethoxy)ethoxy]acetyl), desB27, desB30 human insulin

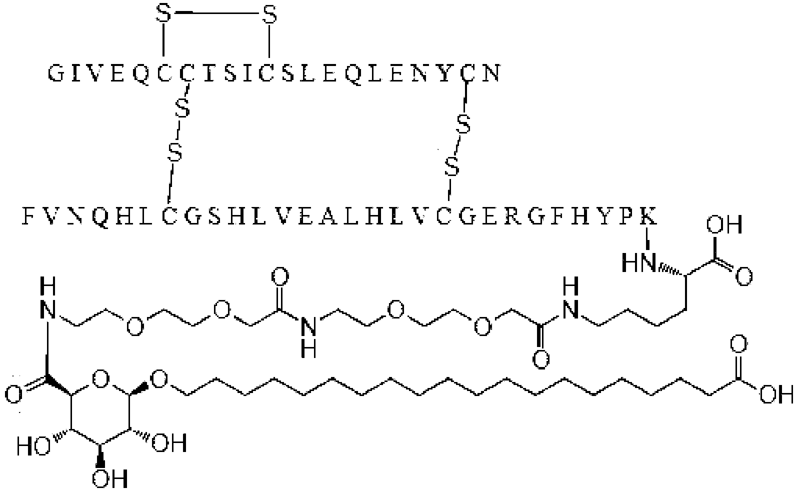

Compound 3 was prepared according to the method described in Example 1, and Fmoc-γ Glu(OtBu)-OH and Fmoc-Thr(tBu)-OH were removed in Table 5. All other materials and preparation methods remain unchanged.

LC-MS: m/z=1579.3[M+4H]⁴⁺, consistent with theoretical values.

### Example 4: Preparation of Compound 4

### The molecular formula and structural formula of Compound 4 are as follows:

A14E, B16H, B25H, B29K (N^{ε}-[1-(19-Carboxyl-nonadecyloxy)-β-D-glucuronyl]-γGlu-[2-(2-{2-[2-(2-Aminoethoxy) ethoxy]acetylamino}ethoxy)ethoxy]acetyl), desB27, desB30 human insulin;

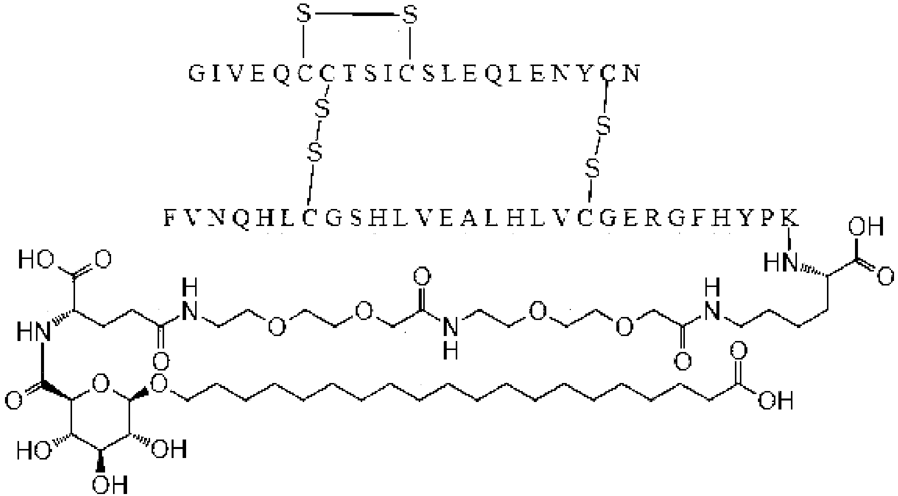

Compound 4 was prepared according to the method described in Example 1, with Fmoc-Thr(tBu)-OH removed as listed in Table 5. All other materials and preparation methods remained unchanged.

LC-MS: m/z=1611.6 [M+4H]⁴⁺, consistent with theoretical values.

Compound 1, Compound 2, Compound 3, and Compound 4 were given by the present invention are shown in Table 9.

**Table 9 Insulin Derivatives and Their Molecular Weights and Purities**

| Name | Molecular Formula | Molecular Weight | Purity (%) |
|---|---|---|---|
| Icodec | C₂₈₀H₄₃₅N₇₁O₈₇S₆ | 6380.33 | 97.68 |
| Compound 1 | C₂₈₁H₄₃₈N₇₀O₈₉S₆ | 6413.36 | 97.36 |
| Compound 2 | C₂₈₆H₄₄₅N₇₁O₉₂S₆ | 6542.47 | 97.89 |
| Compound 3 | C₂₇₇H₄₃₁N₆₉O₈₇S₆ | 6312.25 | 98.50 |
| Compound 4 | C₂₇₇H₄₃₁N₆₉O₈₇S₆ | 6441.37 | 99.08 |

### Experimental Example 1: Study on the Glucose Uptake Capacity of Adipocytes by the Compounds of the Present Invention

### 1.Adipogenic Induction of 3T3-L1 Mouse Embryonic Fibroblasts

1) Cell Plating: Adipocytes were seeded in a 96-well cell culture plate at a density of 4×10⁴cells/well with 200 µL/well of maintenance medium 1 (DMEM, 1% **penicillin-streptomycin,** 10% fetal bovine serum, 1 µg/mL human insulin). Culture in 10% CO₂, 37°C cell incubator.
2) After two days, switch to standard medium 2 (DMEM, 1% double antibodies, 10% fetal bovine serum) and continue the culture.
3) After two days, the cells exhibit an adipocyte phenotype and are ready for glucose uptake assays in adipocytes.

### 2.Procedure for Adipocyte Glucose Uptake Assay

1) KRPH Buffer Preparation: 5 mM Na₂HPO₄, 20 mM HEPES, 1 mM MgSO₄, 1 mM CaCl₂, 136 mM NaCl, 4.7 mM KCl, pH 7.40.
2) On the day of the assay, the adipocyte culture medium is replaced with serum-free DMEM medium 3 and the cells were starved for 2 hours.
3) Sample Dilution: All sample storage solutions were diluted with KRPH buffer solution. Icodec, Compound 1, and Compound 2 were subjected to 3-fold serial gradients dilution with a starting concentration of 25 µM for 10 points.
4) Adipocytes post-starvation were processed using the Well Vario liquid workstation. After a rinse with KRPH buffer, 50 µL of gradient-diluted samples at varying concentrations were added to each well, with duplicate wells for each concentration point. Incubation was performed for 10 minutes.
5) 50 µL/well of KRPH buffer containing 0.25 µCi [³H]-deoxyglucose and 50 µM deoxyglucose were added then incubated for 20 minutes in a CO₂ incubator.
6) Cells were rinsed three times with DPBS containing 10 mM glucose pre-chilled to 4°C, using 200 µL per well for each wash.
7) The adipocytes were lysed with 100 µL of 10% sodium hydroxide solution per well, and then were shaken on an shaker for 20 minutes.
8) The cleaved sample was transferred to the scintillation vial, 2 mL of scintillation liquid was added, and scintillation counting was performed using the Tri-Carb.

Finally, data were analyzed using GraphPad Prism 7.

**Table 1.1 Average EC₅₀ Values of Insulin Derivatives**

| Name | EC₅₀ (nM) |
|---|---|
| Icodec | 193.51 |
| Compound 1 | 174.60 |
| Compound 2 | 128.50 |

| | |
|---|---|
| Note: Each of the above compounds has undergone at least three independent tests. | |

Results Analysis: it clearly shown in the table that Compound 1 and Compound 2 are superior to Icodec in terms of enhancing the glucose uptake capacity of adipocytes.

### Experimental Example 2: Human Serum Albumin (HSA) Binding Study

### 1. Studying the in vitro binding of insulin derivatives with human serum albumin (HSA) through activation of Insulin Receptor B (IRB) phosphorylation.

Cells were seeded at a density of 10,000 cells per well into a 384-well cell culture plate using medium containing 5% serum. The cells were incubated overnight in a 37°C incubator with 5% CO₂. Icodec, Compound 1, and Compound 2 were dissolved and diluted in F12 medium containing 0.1% HSA and 0.1% Casein, respectively. The starting concentration for the test was 60 µM, with all samples subjected to 4-fold serial gradients dilution across 10 points. The cell plates were removed from the incubator, the medium was centrifuged off using a plate washer, the diluted sample was immediately added, and the mixture was allowed to react at room temperature for 5 minutes. The treated cell samples were discarded by using a plate washer, cell lysis buffer solution was immediately added, and the mixture was shaken thoroughly on a shaker to lyse. The cell lysate was diluted with 1× cell lysis buffer. 10 µL of diluted cell lysate was transferred to an Optiplate 384-well plate. The Acceptor Beads mixture was added, the plate was sealed with aluminum foil, the mixture was mixed evenly and stand for incubation at room temperature,then the Donor Beads mixture (protected from light) was added, the mixture was mixed evenly and stand for incubation at room temperature. The plate was read using the Envision instrument in AlphaScreen mode. Finally, the data was analyzed using GraphPad Prism 7.

**Table 2.1 Comparison of the Effects of Casein and HSA on Insulin Derivative-Activated IRB Phosphorylation**

| Name | 0.1% HSA | 0.1% Casein | Ratio of 0.1% HSA EC₅₀ (nM) to 0.1% Casein EC₅₀ (nM) |
|---|---|---|---|
| | EC₅₀(nM) | EC₅₀(nM) | |
| Icodec | 1457.15 | 244.35 | 5.96 |
| Compound 1 | 2578.25 | 296.58 | 8.69 |
| Compound 2 | 2247.00 | 276.63 | 8.12 |

| | | | |
|---|---|---|---|
| Note: Each of the above compounds has undergone at least three independent tests. | | | |

Results Analysis: As shown in the table above, compared with Icodec, Compound 1 and Compound 2 exhibit stronger tight junction to HSA.

Note: In the detection method described in this invention, 0.1% human serum albumin (HSA) is commonly added during the insulin derivative-activated IRB phosphorylation assay. Fatty-acylated insulin can bind tightly to HSA, which affects the results by artificially reducing the apparent efficacy of the compounds. Using 0.1% casein instead can circumvent this issue. The improvement observed after casein administration can be regarded as an indicator of the relative tight junction with which the compound binds to serum albumin.

### 2.Determination of the in vitro binding of insulin derivatives to HSA via SPR

Binding of Icodec, Compound 2, Compound 3, and Compound 4 to human serum albumin on the Biacore 8K instrument.

### 2.1 Experimental Steps

### 2.1.1 Protein-coupled

According to the instructions for the Amine Coupling Kit, Type 2 (Cytiva), immobilize human serum albumin onto the surface of the CM5 sensor chip of the S Series. The specific steps are as follows:
a) EDC and NHS were mixed in a ratio of 1:1. Human serum albumin (HSA) was diluted to 20 µg/mL with acetic acid solution of pH 4.5. The EDC/NHS mixture solution, HSA protein solution, and 1M ethanolamine-hydrochloric acid solution were added to the detection plate according to the program settings.
b) The detection plate was placed into the Biacore 8K sample compartment. The EDC/NHS mixture solution, HSA protein solution, and 1M ethanolamine-hydrochloric acid solution were sequentially injected into the channels on the surface of the CM5 chip by the Biacore 8K according to the program. Through this process, the chip was activated, the ligand was coupled, and the chip was deactivated. Each channel consists of two circulation pools, with circulation pool 1 serving as the reference channel undergoing only surface activation and deactivation without injection of HSA protein solution. Specific parameters were as follows:

**Table 2.2 Ligand Coupling Parameters**

| Activation and Blocking Time | Ligand Binding Time | Flow Rate |
|---|---|---|
| 420 s | ~150 s | 10 L/min |

### 2.1.2 Sample Testing

a) The stock solutions of Icodec, Compound 2, Compound 3, and Compound 4 were diluted by running PBST buffer solution (137 mM NaCl, 2.7 mM KCl, 8 mM Na₂HPO₄, 2 mM KH₂PO₄, 0.05% Tween-20). All samples were started at 200 µM and were subjected to six points of 2-fold serial continuous gradient dilution.
b) The prepared samples Icodec, Compound 2, Compound 3, Compound 4, and running buffer were transferred to the detection plate according to the program. The detection plate was placed into the sample compartment of the Biacore 8K, with the running buffer serving as the 0-concentration reference sample.
c) Run the detection program, with samples sequentially injected into each channel of the chip according to the program. Specific parameters are as follows:

**Table 2.3 Sample Testing Conditions**

| Project | Combined Time | Dissociation Time | Flow Rate |
|---|---|---|---|
| Sample Testing Conditions | 60 s | 180 s | 30µL/min |

### 2.1.3 Data Analysis

Before analyzing raw data, it must be processed according to the following procedure:
a) Set the start time for all concentration compounds and buffer solution injections to 0 seconds.
b) Set the baseline response value for all concentrations and all channels to 0 RU.
c) Subtract the corresponding reference channel response value from the test channel response value for each channel to give the response values for samples of different concentrations.
d) The final sensing map is given by subtracting the response value of the zero-concentration sample from the response values of samples at different concentrations.

Finally, the data was analyzed using BiaCore Insight Evaluation Software, employing a 1:1 binding model for analysis.

**Table 2.4 Binding Kinetics of Insulin Derivatives with HSA**

| Name | ka (1/Ms) | kd (1/s) | KD (M) |
|---|---|---|---|
| Icodec | 2.39E+02 | 1.12E-01 | 4.68E-04 |
| Compound 2 | 4.99E+02 | 1.80E-01 | 3.61E-04 |
| Compound 3 | 9.95E+02 | 1.43E-01 | 1.44E-04 |
| Compound 4 | 6.12E+02 | 1.69E-01 | 2.75E-04 |

Results Analysis: The table clearly shows that Compound 2, Compound 3, and Compound 4 exhibit superior binding affinity to HSA compared to Icodec.

### Experimental Example 3: Half-life of insulin derivatives

6 healthy male Beagle dogs, aged 8-9 months and weighing 7-10 kg, were selected from the reserve animal group. During the experiment, animals were housed individually with free access to food and water.

After one week of adaptive feeding, the experiment commenced. Beagles were divided into two treatment groups based on body weight, with three dogs per group. The administered dose was 4 nmol/kg for both groups. Icodec and Compound 2 were administered intravenously, respectively, in a solvent comprising: 5 mM disodium hydrogen phosphate, 140 mM sodium chloride, and 70 ppm Tween 20, and the pH of the solvent was 8.0.

Icodec and compound 2 were dissolved separately in solvent to a concentration of 40 nmol/mL. The dosing volume was 0.1 mL/kg. Single intravenous administration. Blood samples were collected at 0.05h, 0.167h, 0.5h, 1h, 2h, 4h, 6h, 8h, 24h, 32h, 48h, 72h, 96h, 144h, 192h, and 216h post-administration to determine their plasma concentrations of administered compound. Animals were fasted at sampling times; otherwise, they had free access to food and water. Finally, the data was analyzed using Phoenix WinNonlin 6.3.

**Table 3.1 Half-Life of Insulin Derivatives Following Intravenous Injection in Normal Beagles**

| Compounds | Icodec | Compound 2 |
|---|---|---|
| T_{1/2} (h) | 40.6 | 45.8 |
| **AUC_{0-inf} (ng.h/mL)** | / | 11788.0 |

Results Analysis: The table clearly demonstrates that in the intravenous administration trial on normal beagles, Compound 2 exhibits a superior half-life compared with Icodec.

### Experimental Example 4: Pharmacodynamic Study in Normal SD Rats

Normal male SD rats aged 8-9 weeks, body weight 280-320 g, were selected. Animals were housed in animal housing facilities with strictly controlled environmental conditions, where the temperature is maintained between 20°Cand 24°C and humidity was kept between 30 and 70%. During the experiment, animals were housed individually with free access to food and water.

After one week of adaptive feeding, the experiment commenced. Rats were assigned to Solvent group 1, Solvent group 2, and the treatment group based on body weight and blood glucose levels before administration, with eight rats per group. The administered doses were all 650 nmol/kg. Subcutaneous injection of solvent, or subcutaneous injections of Icodec, Compound 2, Compound 3, and Compound 4, respectively, wherein the solvents described in Solvent Group 1 and Solvent Group 2 comprise: 30 mM phenol, 1.6% (w/v) glycerol, and the pH of the solvent is 8.0.

The above drugs were dissolved in the solvent to a concentration of 216.7 nmol/mL. The dosing volume is 3 mL/kg. For a single subcutaneous administration, with the administration time set as 0 hours, blood glucose levels were measured at 0 h before dosing, and at 4 h, 8 h, 24 h, 28 h, 48 h, 72 h, 96 h, and 120 h after dosing (without dietary restrictions). The dose-response curve of blood glucose over time for each single dose of insulin derivative was plotted.

To demonstrate the effect of the insulin derivative of the present invention on blood glucose levels, the area under the blood glucose-time curve (AUC) was calculated for each individual dose-response curve from 0 to the monitoring endpoint. The smaller the AUC value, the better the hypoglycemic effect and the more effective the drug.

**Table 4.1 Effects of Single Administrations of Icodec and Compound 2 on Blood Glucose Levels in SD Rats (Mean ± SEM, n = 8)**

| Groups | Time Points (h) | | | | | | |
|---|---|---|---|---|---|---|---|
| | 0 | 4 | 24 | 48 | 72 | 96 | 120 |
| Solvent Group 1 | 7.0±0.1 | 7.3±0.2 | 7.5±0.1 | 7.0 ± 0.1 | 7.6±0.1 | 7.1±0.1 | 7.3±0.1 |
| Icodec | 7.2±0.2 | 4.8±0.4** | 6.2±0.3 | 7.0±0.1 | 7.5±0.2 | 7.0±0.2 | 7.5±0.1 |
| Compound 2 | 6.9±0.1 | 3.9±0.4** | 5.4±0.5* | 5.0±0.1* | 6.4±0.6 | 7.2 ± 0.1 | 7.5±0.1 |

| | | | | | | | |
|---|---|---|---|---|---|---|---|
| Note:*P<0.05, **P<0.01 vs. Solvent Group 1 | | | | | | | |

**Table 4.2 Effects of Single Administrations of Icodec, Compound 3, and Compound 4 on Blood Glucose Levels in SD Rats (Mean ± SEM, n = 8)**

| Groups | Time Points (h) | | | | | | |
|---|---|---|---|---|---|---|---|
| | 0 | 4 | 24 | 48 | 72 | 96 | 120 |
| Solvent Group 2 | 7.4±0.1 | 7.3±0.1 | 7.3 ± 0.2 | 7.5±0.2 | 7.6±0.1 | 7.6±0.2 | 7.3±0.1 |
| Icodec | 7.5±0.1 | 4.1±0.3** | 4.0±0.4** | 6.2 ± 0.5 | 7.8±0.2 | 7.8±0.1 | 7.5±0.1 |
| Compound 3 | 7.5 ± 0.1 | 3.7±0.3** | 3.6±0.4** | 4.8±0.4** | 7.2±0.1 | 7.7±0.2 | 7.7±0.1 |
| Compound 4 | 7.3 ± 0.1 | 3.6±0.3** | 4.2±0.4** | 4.4±0.5** | 6.6±0.5* | 7.8±0.1 | 7.5 ± 0.1 |

| | | | | | | | |
|---|---|---|---|---|---|---|---|
| Note:*P<0.05, **P<0.01 vs. Solvent Group 2 | | | | | | | |

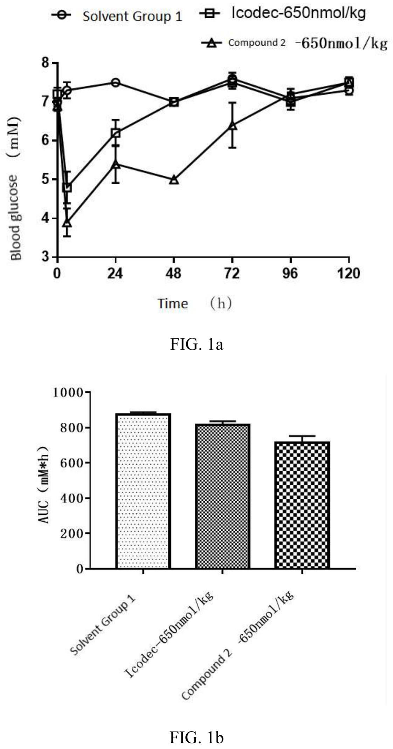

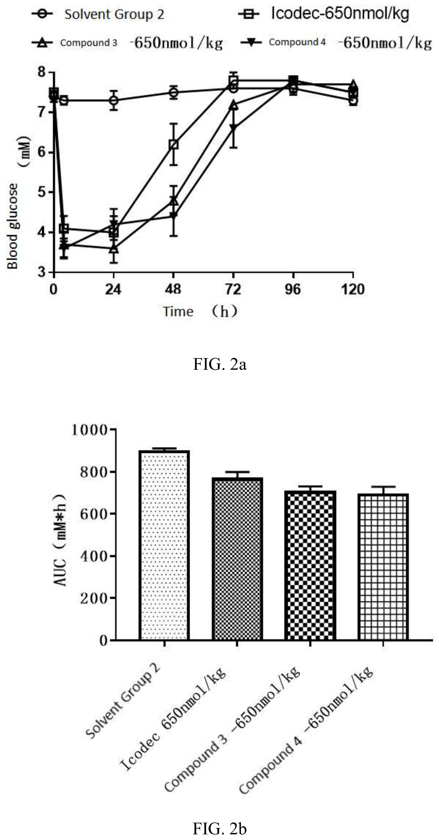

As shown in Table 4.1, Figures 1a and 1b, Table 4.2, Figures 2a and 2b, the hypoglycemic effect of the insulin derivatives Compound 2, Compound 3, and Compound 4 of the present invention are superior to Icodec.

### Experimental Example 5: Pharmacodynamic Study of Normal Beagles

12 healthy male Beagle dogs aged 8-9 months, body weight 7-10 kg, were selected from the reserve **animal group.** During the experiment, animals were housed individually with free access to food and water.

After one week of adaptive feeding, the experiment commenced. Beagles were assigned to either the solvent group or the treatment group based on body weight and blood glucose levels before administration, with four dogs in each group. The administered dose was all 21 nmol/kg. Intravenous injection of the solvent, or intravenous injections of Icodec and Compound 2, respectively, wherein the solvent comprises: 5 mM disodium hydrogen phosphate, 140 mM sodium chloride, 70 ppm Tween 20, and the pH of the solvent is 8.0.

The above drugs were dissolved in the solvent to a concentration of 210 nmol/mL. The dosing volume was 0.1 mL/kg. Single intravenous administration, with the administration time set to 0 point. Blood glucose levels were measured at 0 h before administration, and at 2 h, 24 h, 48 h, and 72 h after administration. Animals were fasted at sampling times; otherwise, they had free access to food and water. The dose-response curve of blood glucose over time for each single dose of insulin derivatives were plotted.

To demonstrate the effect of the insulin derivative of the present invention on blood glucose levels, the area under the blood glucose-time curve (AUC) was calculated for each individual dose-response curve from 0 to the monitoring endpoint. The smaller the AUC value, the better the hypoglycemic effect and the more effective the drug.

**Table 5.1 Effect of Single Administration on Blood Glucose Levels in Beagles (Mean ± SEM, n = 4)**

| Groups | Time Points (h) | | | | |
|---|---|---|---|---|---|
| | 0 | 2 | 24 | 48 | 72 |
| Solvent | 4.2±0.4 | 4.2±0.2 | 4.6±0.3 | 4.3±0.3 | 4.3±0.3 |
| Icodec | 4.4±0.1 | 3.0±0.2* | 3.4±0.3** | 3.2±0.3* | 3.9±0.1 |
| Compound2 | 4.4±0.1 | 2.6±0.3* | 2.6±0.5** | 2.9±0.2** | 3.6±0.3 |

| | | | | | |
|---|---|---|---|---|---|
| Note: *P<0.05, **P<0.01 vs. Solvent Group. | | | | | |

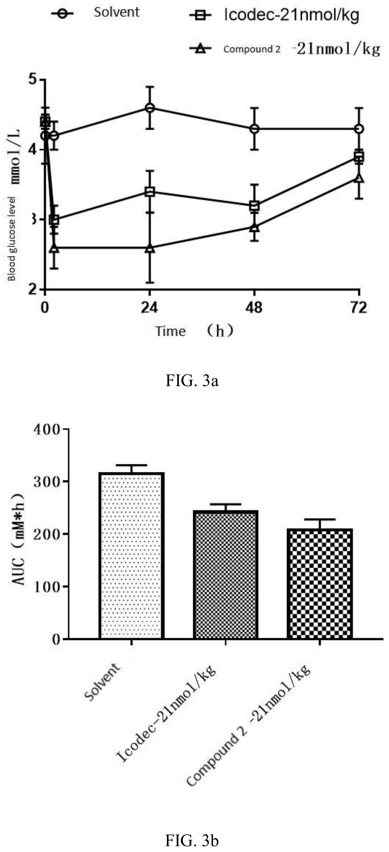

As shown in Table 5.1, Figures 3a and 3b, the hypoglycemic effect of the insulin derivative Compound 2 of the present invention is superior to Icodec.

## Claims

1. An insulin derivative of formula (I), or salts or solvates thereof: 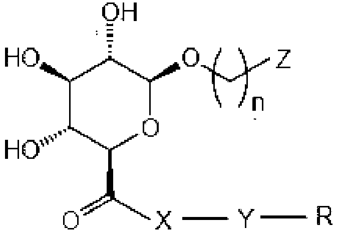 wherein,
Z is CH₃ or COOH;
n is 17,18,19,20 or 21;
X is yGlu or absent;
Y is -NH-(CH₂)₂-O-(CH₂)₂-O-CH₂-CO-NH-(CH₂)₂-O-(CH₂)₂-O-CH₂-CO-, or absent;
R is the insulin parent body, wherein the ε-amino group of the lysine side chain at position 29 of the B-chain is connected to Y via an amide bond.

2. The derivative according to claim 1, **characterized in that**, the insulin parent body is selected from: A14E, B16H, B25H, desB30 human insulin; A14E, B16H, B25H, desB27, desB30 human insulin.

3. The derivative according to claim 1, **characterized in that** , the insulin derivative of formula (I) is selected from:
Compound 1: A14E, B16H, B25H, B29K (N^{ε}-[1-(19-Carboxyl-nonadecyloxy)-β-D-glucuronyl]-[2-(2-{2-[2-(2-Aminoethoxy)ethoxy]acety lamino}ethoxy)ethoxy]acetyl), desB30 human insulin;
Compound 2: A14E, B16H, B25H, B29K (N^{ε}-[1-(19-Carboxyl-nonadecyloxy)-β-D-glucuronyl]-γGlu-[2-(2-{2-[2-(2-Aminoethoxy) ethoxy]acetylamino}ethoxy)ethoxy]acetyl), desB30 human insulin;
Compound 3: A14E, B16H, B25H, B29K (N^{ε}-[1-(19-Carboxyl-nonadecyloxy)-β-D-glucuronyl]-[2-(2-{2-[2-(2-Aminoethoxy)ethoxy]acety lamino}ethoxy)ethoxy]acetyl), desB27, desB30 human insulin;
Compound 4: A14E, B16H, B25H, B29K (N^{ε}-[1-(19-Carboxyl-nonadecyloxy)-β-D-glucuronyl]-yGlu-[2-(2-{2-[2-(2-Aminoethoxy) ethoxy]acetylamino}ethoxy)ethoxy]acetyl), desB27, desB30 human insulin.

4. The derivative according to claim 1, **characterized in that** , the insulin derivative of formula (I) is selected from:
Compound 2: A14E, B16H, B25H, B29K (N^{ε}-[1-(19-Carboxyl-nonadecyloxy)-β-D-glucuronyl]-γGlu-[2-(2-{2-[2-(2-Aminoethoxy) ethoxy]acetylamino}ethoxy)ethoxy]acetyl), desB30 human insulin.

5. A pharmaceutical composition, **characterized in that**, the pharmaceutical composition comprises an effective amount of the insulin derivative of Formula (I), or salts or solvates thereof, and pharmaceutically acceptable adjuvants, diluents, carriers, or excipients.

6. The pharmaceutical composition according to claim 5, **characterized in that**, the pharmaceutical composition is injectable preparation or lyophilized powder, tablet, pill, lozenge, soft capsule, hard capsule, granule, powder, solution, microneedle, suspension, or syrup.

7. The pharmaceutical composition according to claim 5, **characterized in that**, the pharmaceutical composition is in the form of microcapsule, microsphere, nanoparticle, or liposome.

8. The pharmaceutical composition according to claim 5, **characterized in that**, the pharmaceutical composition is intended for oral administration, inhalation administration, transdermal administration, or parenteral administration, wherein the parenteral administration is selected from intraperitoneal, intramuscular, intra-arterial, intravenous, subcutaneous, or intradermal injection.

9. The pharmaceutical composition according to claim 6, **characterized in that**, the pharmaceutical composition is administered at a frequency of at least once daily, once weekly, or once monthly.

10. The pharmaceutical composition according to claim 5, **characterized in that**, the pharmaceutical composition further comprises: metformin agents, sulfonylureas, SGLT-1/2 inhibitors, DPP-4 inhibitors, insulin and analogs thereof, GLP-1 and analogs thereof, GCG and analogs thereof, GIP and analogs thereof, FGF-21 and analogs thereof, or multi-target drugs among the above drugs, or two or more of them.

11. The use of the insulin derivative of formula (I) or its salt or solvate described in any one of claims 1 to 4, or the pharmaceutical composition described in any one of claims 5 to 10, in the preparation of a medicament for the treatment, prevention, or alleviation of diabetes, type 1 diabetes, type 2 diabetes, impaired glucose tolerance, hyperglycemia, dyslipidemia, or obesity.
